# FASCICULE DE BREVET EUROPEEN

(11) **EP 2 579 940 B1**
(45) Date de publication et mention de la délivrance du brevet: **19.03.2014**
(21) Numéro de dépôt: 11725709.7
(22) Date de dépôt: 08.06.2011
(51) Int. Cl.: H01L 21/02, H01L 21/64, H01L 29/16, H01L 29/45, A61N 1/05, A61N 1/36

(54) **PROCÉDÉ DE FABRICATION D'UN IMPLANT SOUPLE RÉTINIEN INTRAOCULAIRE À ÉLECTRODES EN DIAMANT DOPÉ**
VERFAHREN ZUR HERSTELLUNG EINES FLEXIBLEN INTRAOKULAREN RETINAIMPLANTATS MIT DOTIERTEN DIAMANTELEKTRODEN
METHOD FOR MANUFACTURING A FLEXIBLE INTRAOCULAR RETINAL IMPLANT HAVING DOPED DIAMOND ELECTRODES

(30) Priorité: 09.06.2010 FR 1054550
(43) Date de publication de la demande: 17.04.2013
(73) Titulaire: Commissariat à l'Énergie Atomique et aux Énergies Alternatives, 75015 Paris (FR); Chambre de Commerce et d'Industrie de Paris (Esiee Paris), 93160 Noisy-le-Grand (FR)
(72) Inventeur: SCORSONE, Emmanuel, F-78114 Magny-les-Hameaux (FR); BERGONZO, Philippe, F-91300 Massy (FR); BONNAURON, Mathias, F-91191 Gif-sur-Yvette cedex (FR); LISSORGUES, Gaëlle, F-94170 Le Perreux-sur-Marne (FR); ROUSSEAU, Lionel, F-94170 Le Perreux-sur-Marne (FR)
(74) Mandataire: Ilgart, Jean-Christophe
(86) Numéro de dépôt international: PCT/EP2011/059510
(87) Numéro de publication internationale: WO 2011/154455

(56) Documents cités:
- EP-A1- 1 947 220
- WO-A1-2010/012739
- FR-A1- 2 917 229
- US-A1- 2006 175 953

## Description

### DOMAINE TECHNIQUE

La présente invention a trait au domaine des implants rétiniens dont la fonction est de restaurer la vision de patients rendus aveugles par une maladie dégénérative de la rétine.

L'invention concerne plus particulièrement un procédé de fabrication d'un implant rétinien souple destiné à être implanté dans le globe oculaire d'un patient et dont la fonction est de recevoir et de traiter des stimuli visuels extérieurs qui sont utilisés pour exciter la rétine malade du patient par le biais d'électrodes.

On précise que le procédé selon l'invention peut être appliqué indifféremment à la réalisation d'implants « épi-rétiniens », c'est-à-dire destiné à être disposé sur la rétine du patient, ou à la réalisation d'implants « sous-rétiniens », c'est-à-dire destiné à être placé derrière la rétine.

### ÉTAT DE LA TECHNIQUE ANTÉRIEURE

Aujourd'hui les pathologies de dégénérescence rétinienne, comme par exemple la dégénérescence maculaire liée à l'âge (DMLA), touchent environ 12 millions de personnes aux Etats Unis et en Europe. Certaines études ont même prévu l'accroissement de ce phénomène du fait du vieillissement des populations occidentales : selon les prévisions, en 2012, 1% de la population sera affecté par une pathologie rétinienne à 55 ans, 10% à 65, 25% à 75 et jusqu'à 60% à 90 ans.

Parmi les pathologies rétiniennes les plus fréquentes, la rétinite pigmentaire et la DMLA conduisent à une dégénération progressive des photorécepteurs, conduisant à la perte progressive d'une vision localisée sur la rétine, alors que l'ensemble du système neuronal des couches rétiniennes est encore intact et apte à acheminer une information électrique vers le cerveau.

Afin de restituer la vision aux personnes atteintes par ces maladies dégénératives de la rétine, il est connu d'implanter sur la rétine des prothèses rétiniennes qui viennent directement stimuler électriquement les couches encore saines du tissu rétinien.

A ce propos, l'art antérieur fait aujourd'hui état de géométries d'implants très variées, dont la structure commune est formée d'un ensemble d'électrodes de taille micrométrique (c'est-à-dire comprise entre 1 et 1000 µm), également appelées « microélectrodes », dont la base est disposée sur un substrat support en matériau électriquement isolant, ces électrodes étant réalisées en un matériau électriquement conducteur.

Certains de ces implants sont composés d'électrodes métalliques disposées sur un substrat rigide, par exemple en silicium (voir le document [1] référencé à la fin de la description). Or, ce type d'implants présente un certain nombre d'inconvénients. Tout d'abord, leur rigidité peut endommager les tissus rétiniens lors de leur implantation sur ou sous la rétine. Dans certains cas, l'électrode peut en outre être cassante. De plus, le manque de souplesse de ces implants ne permet pas d'obtenir un positionnement adéquat des implants et en particulier d'épouser correctement la surface des tissus à stimuler, ce qui peut potentiellement empêcher certaines électrodes de venir en contact avec les zones d'intérêt à stimuler.

Pour résoudre ce problème de rigidité, des implants comportant des microélectrodes disposées sur des substrats souples ont été développés. Les substrats souples de ces implants sont généralement fabriqués à partir de polymères de type polyimide ou pyralène, qui sont des matériaux particulièrement intéressants pour leur flexibilité, ainsi que pour leur inertie chimique et leur biocompatibilité (voir le document [2]).

Les matériaux utilisés pour les électrodes des implants sont, quant à eux, généralement des métaux ou des oxydes métalliques, tels que l'or, le platine ou l'oxyde d'iridium. Or, un des inconvénients des électrodes fabriquées à base de ces matériaux est qu'elles souffrent de corrosion et d'un manque de stabilité mécanique et chimique, en particulier en milieu in-vivo, qui est un milieu relativement hostile pour les électrodes métalliques. D'autre part, ce phénomène de vieillissement des électrodes est accéléré par le passage d'un courant à travers les électrodes lorsque celles-ci sont utilisées pour la stimulation. En effet, l'utilisation d'électrodes métalliques dans les implants impose l'application de niveaux de courants de stimulation élevés, ce qui induit, à long terme, un vieillissement prématuré des électrodes et nécessite une intervention chirurgicale lourde afin de remplacer l'implant usagé.

Par ailleurs, il est avantageux d'avoir une grande densité d'électrodes sur l'implant pour rétablir la meilleure acuité visuelle possible. Pour ce faire, la taille de chaque électrode doit être la plus petite possible et les électrodes doivent être rapprochées au maximum, ce qui pose un certain nombre de problèmes.

Tout d'abord, la diminution de la taille des électrodes entraine une perte de leur capacitance. Pour résoudre ce problème, il est nécessaire de structurer la surface des électrodes afin d'en augmenter la surface spécifique, ce qui est très difficile à réaliser sur des électrodes métalliques. Une solution possible à ce problème est de réaliser les électrodes métalliques avec du noir de platine, c'est-à-dire avec un empilement de particules de platine de tailles nanométriques. Cependant, la tenue mécanique de ce type d'électrodes est très mauvaise, en particulier lorsqu'elles sont en contact avec des tissus vivants.

D'autre part, le rapprochement des électrodes peut provoquer des interférences entre les électrodes adjacentes et conduire ainsi à une perte de résolution de l'implant. Une façon de réduire ces interférences éventuelles est d'appliquer moins de courant aux électrodes. Cependant, en réduisant le courant, les électrodes ne peuvent alors stimuler efficacement les cellules neuronales qu'à condition que ces cellules soient les plus proches possible des électrodes, voire en contact direct avec elles. Pour cela, les électrodes doivent être constituées à base d'un matériau biocompatible. Un matériau biocompatible est un matériau qui ne provoque pas de nécrose des tissus environnant et qui possède en outre une grande affinité avec les cellules à stimuler, c'est-à-dire qui est favorable à l'accroche de cellules neuronales directement sur sa surface. Il est connu par exemple que, dans le cas des implants rétiniens, lorsque des électrodes métalliques sont utilisées, il y a formation d'une couche de cellules gliales qui s'intercale entre la surface de l'électrode et les tissus neuronaux, ce qui affectent la transmission électrique entre les neurones et l'électrode.

Dans ce contexte, les gens du métier ont donc cherché des matériaux alternatifs aux matériaux métalliques, présentant une meilleure stabilité et une meilleure affinité avec les tissus vivants. C'est ainsi qu'ont été proposées des électrodes à base de nanotubes de carbone, qui présentent potentiellement une meilleure stabilité et une meilleure affinité avec les tissus vivants que les électrodes métalliques. Cependant, les électrodes à base de nanotubes sont très fragiles et ont tendance à se casser au contact des tissus biologiques et se décrochent, par frottement, du substrat support sur lequel elles sont disposées.

Les inventeurs ont, quant à eux, proposé de réaliser des implants à base d'électrodes en diamant dopé disposées sur un substrat souple en matériau biocompatible et électriquement isolant tel que le polyimide (document [3]). En effet, comme le diamant est constitué de carbone, il possède une biocompatibilité bien meilleure que n'importe quel métal. Aussi, en créant des électrodes en diamant dopé, on peut espérer limiter les réactions de défense du corps vis-à-vis de l'implant (comme, par exemple, les réactions gliales), obtenant de ce fait un meilleur contact entre les tissus vivants et l'implant en favorisant l'absence de rejet de l'implant. En améliorant la biocompatibilité, on peut également espérer réduire les courants de stimulation et ainsi augmenter le nombre de pixels et, au final, améliorer la définition de l'image recréée grâce à l'implant.

Cependant, la croissance du diamant est généralement réalisée dans un réacteur CVD (initiales de « Chemical Vapour Déposition » en anglais) par une technique de croissance diamant assisté par plasma micro-ondes ou RadioFréquences ou par filament chaud, bien connue des experts du domaine de la croissance de diamant synthétique. La croissance du diamant est ainsi obtenue par dépôt de vapeur chimique dans un plasma en présence d'hydrogène et d'une source de carbone, par exemple le méthane. Le dépôt est donc réalisé à une température comprise typiquement entre 500 et 1000°C. Or, cette échelle de température est incompatible avec la croissance de diamant de synthèse sur les substrats souples en polymères couramment utilisés pour la réalisation d'implants souple. La réalisation de ces implants semblait donc être compromise. Les inventeurs se sont donc fixé comme but d'élaborer un procédé permettant de réaliser de tels implants à substrats souples et à électrodes en diamant dopé.

### EXPOSÉ DE L'INVENTION

L'invention a donc pour objet un procédé de fabrication d'un implant rétinien intraoculaire, destiné à stimuler des cellules de la rétine par l'envoi d'impulsions électriques pour produire une vision artificielle, ledit implant comprenant une plaque souple en un matériau biocompatible et électriquement isolant, qui est munie, sur l'une de ses faces, d'une pluralité d'électrodes en diamant dopé espacées les unes des autres et reliées à des lignes d'interconnexion destinées à conduire un courant électrique jusqu'aux électrodes pour qu'elles puissent transmettre des impulsions électriques auxdites cellules de la rétine, ledit procédé comprenant les étapes successives suivantes :
- fourniture d'un moule apte à supporter la croissance d'une couche de diamant dopé, ledit moule comportant, sur l'une de ses faces, un ensemble d'éléments, qui sont tous en creux ou tous en saillie par rapport à la surface de ladite face, et qui forment une empreinte des électrodes de l'implant que l'on souhaite obtenir ;
- réalisation des électrodes en diamant dopé par croissance d'une couche de diamant dopé dans tout ou partie de l'espace occupé par les éléments de l'empreinte ;
- formation d'une première couche de matériau électriquement isolant sur la face du moule comportant l'empreinte, ladite première couche recouvrant au moins l'espace entre les électrodes, tout en laissant découverte au moins une partie de la surface de chaque électrode ;

- réalisation des lignes d'interconnexion par dépôt d'un matériau électriquement conducteur au moins dans les espaces non recouverts par la première couche de matériau électriquement isolant ;
- formation d'une seconde couche de matériau électriquement isolant sur la face du moule comprenant l'empreinte, ladite seconde couche recouvrant les lignes d'interconnexion et la première et la seconde couche de matériau électriquement isolant formant la plaque souple de l'implant ; et
- retrait du moule.

En procédant ainsi, les électrodes de l'implant sont en saillie (c'est-à-dire en relief) par rapport à la face sur laquelle elles sont disposées.

Il est à noter que la plaque de l'implant est souple, ce qui sous-entend que l'épaisseur de la première et de la seconde couche de matériau électriquement isolant est inférieure, pour des matériaux déterminés, à une valeur seuil que l'homme du métier est capable de déterminer. On précise qu'une plaque souple est une plaque qui est flexible, c'est-à-dire qui présente une déformation en flexion supérieure ou égale à une valeur limite déterminée lorsqu'une pression déterminée est appliquée sur la surface de cette couche.

Le substrat souple est de préférence un polymère choisi pour ses propriétés mécaniques adéquates, ainsi que pour sa biocompatibilité et sa biostabilité. Ce polymère sera par exemple un polymère siliconé, un polyimide ou un pyralène.

Avantageusement, les matériaux de la première et de la seconde couche sont un même matériau.

Avantageusement, le retrait du moule est obtenu par élimination chimique ou mécanique du moule. L'élimination peut, par exemple, consister en une dissolution dans un acide adéquat ou un ponçage mécanique. Le moule peut également être détaché par décollement. Par exemple, le moule peut être un substrat en silicium ou en verre, car ces matériaux peuvent être dissous dans un mélange adéquat d'acides nitrique et fluorhydrique pour le premier, ou dans l'acide fluorhydrique seul, pour le second.

Selon une variante, le procédé comprend en outre, entre l'étape de fourniture d'un moule et l'étape de réalisation des électrodes, une étape de dépôt, sur la face du moule comportant l'empreinte, d'une couche sacrificielle supportant la croissance d'une couche de diamant dopé, ladite couche sacrificielle étant destinée à être retirée lors de l'étape de retrait du moule. Cette couche sacrificielle a alors pour fonction de faciliter le retrait du moule. Dans ce cas, le retrait du moule est avantageusement obtenu par dissolution de la couche sacrificielle ou en détachant la couche sacrificielle. Le détachement de la couche sacrificielle est possible lorsque le matériau de la couche sacrificielle est choisi de sorte qu'il possède un coefficient d'adhésion sur le diamant dopé et sur la première couche de matériau électriquement isolant qui est inférieur au coefficient d'adhésion de la couche sacrificielle sur le moule. Quant à la dissolution de la couche sacrificielle, elle peut être obtenue en utilisant un solvant adéquat (acide...). Par exemple, il est possible de déposer une couche d'oxyde de silicium sur un substrat de silicium ; la couche d'oxyde sera gravée en fin de procédé dans une solution d'acide fluorhydrique. Alternativement, la couche sacrificielle peut aussi être une couche métallique qui sera dissoute par électroérosion.

On précise que, comme la couche sacrificielle est destinée à être retirée, il suffit qu'elle supporte la croissance du diamant dopé et peut indifféremment être en un matériau électriquement isolant ou conducteur.

Avantageusement, les électrodes ont une forme allongée avec une hauteur qui est au moins deux fois plus grande que la base, la base étant la partie de l'électrode en contact avec la couche de premier matériau électriquement isolant. Les électrodes peuvent ainsi être des éléments en forme de colonnes, d'aiguilles ou de pointes. Avantageusement, la hauteur des électrodes est calculée de sorte que l'extrémité des électrodes puisse atteindre les cellules rétiniennes que l'on souhaite stimuler.

De préférence, les électrodes sont disposées perpendiculairement à la surface de la plaque souple.

Les cellules de la rétine qu'il est possible de stimuler à l'aide des électrodes peuvent être des ganglions, des cellules bipolaires, des neurones...

Avantageusement, les électrodes sont disposées selon un motif qui reflète la disposition des cellules de la rétine que l'on souhaite stimuler.

Avantageusement, les électrodes forment un réseau. De préférence, ce réseau est à pas régulier.

Avantageusement, la plus grande étendue des électrodes mesurée dans une direction parallèle au plan de la face de la plaque souple comportant les électrodes est de dimension micrométrique ou inférieure. Cette étendue est, de préférence, nanométrique, c'est-à-dire comprise entre 1 et 1000 nm.

Avantageusement, la distance qui sépare deux électrodes adjacentes est de dimension micrométrique ou inférieure. De préférence, cette distance est nanométrique.

Le dopage du diamant permet de rendre le matériau électriquement conducteur, afin de pouvoir l'utiliser pour véhiculer les signaux électriques qui serviront à la stimulation cellulaire. Le diamant peut être dopé avec n'importe quel matériau et en des quantités suffisantes pour rendre le diamant dopé électriquement conducteur. De préférence, le diamant dopé est du diamant dopé au bore. Il est en effet connu que le diamant dopé au bore possède d'excellentes propriétés électrochimiques, de biocompatibilité et de stabilité mécanique, ce qui fait de lui un matériau idéal pour la réalisation des électrodes de notre implant. De préférence, la concentration en bore dans le diamant dopé est comprise entre 10¹⁸ et 10²¹ atomes par cm³.

La fourniture du moule peut être obtenue de différentes manières.

Selon une première variante, la fourniture du moule comprend les étapes successives suivantes :
- fourniture d'un substrat apte à supporter la croissance d'une couche de diamant dopé sur une de ses faces ;
- dépôt d'une couche de poudre de diamant sur ladite face du substrat, les grains de la poudre de diamant ayant une taille nanométrique ;
- dépôt d'une couche de résine photosensible d'une épaisseur déterminée sur la couche de poudre de diamant ;
- structuration de la couche de poudre de diamant par photolithographie de la couche de résine photosensible pour obtenir l'empreinte du moule.

Selon une seconde variante, la fourniture du moule comprend les étapes successives suivantes :
- fourniture d'un substrat apte à supporter la croissance d'une couche de diamant dopé sur une de ses faces ;
- dépôt d'une couche de poudre de diamant sur ladite face du substrat, les grains de la poudre de diamant ayant une taille nanométrique ;
- dépôt d'une couche de résine photosensible d'une épaisseur déterminée sur la couche de poudre de diamant ;
- structuration de la couche de résine photosensible par photolithographie jusqu'à atteindre la couche de poudre de diamant ;
- dépôt d'une couche métallique sur la face du substrat, ladite couche métallique recouvrant la couche de résine structurée et la couche de poudre de diamant non masquée par la couche de résine structurée ;
- retrait de la couche de résine structurée de manière à former une couche métallique structurée ;
- structuration de la couche de poudre de diamant par gravure de la couche de poudre de diamant non masquée par la couche métallique structurée ;
- retrait de la couche métallique structurée pour obtenir l'empreinte du moule. On obtient ainsi un substrat comportant une couche structurée en nanopoudres de diamant.

La formation de la couche métallique structurée peut être obtenue par un procédé de lift-off.

La structuration de la couche de poudre de diamant peut se faire par gravure plasma de la couche de poudre de diamant non protégée par la couche métallique structurée.

Selon une troisième variante, le moule est obtenu en gravant localement la face d'un substrat apte à supporter la croissance d'une couche de diamant dopé de manière à réaliser un moule ayant la géométrie inverse de celle des électrodes désirées, c'est-à-dire, en d'autres termes, formant l'empreinte négative des électrodes. Pour effectuer la structuration de la face du substrat et ainsi réaliser le moule, on peut utiliser tous les moyens de gravure liquide ou sèche connus de l'homme du métier. En ce qui concerne le choix des matériaux aptes à supporter la croissance du diamant dopé, il est préférable de choisir des matériaux qui peuvent facilement être gravés, comme le silicium ou le verre. Dans un substrat de silicium, les cavités peuvent être réalisées par des techniques connues de gravure localisée telle que la RIE (« Reactive Ion Etching ») ou encore la DRIE (« Deep Reactive Ion Etching »). Des gravures en solutions liquides peuvent aussi être réalisées, dans des solutions à base d'acide fluoridrique, pour le verre, ou dans des solutions à base d'hydroxyde de potassium (KOH), pour le silicium.

La croissance du diamant de synthèse dopé est, quant à elle, obtenue par une méthode de croissance CVD (« Chemical Vapour Deposition » en anglais) : la croissance du diamant dopé est réalisée dans un réacteur CVD de croissance assisté par plasma micro-ondes ou RadioFréquences ou par filament chaud, technique bien connue des experts du domaine de la croissance de diamant synthétique. La croissance se fait par dépôt de vapeur chimique dans un plasma en présence d'hydrogène, d'une source de carbone, par exemple le méthane, et d'une source de dopant. Dans le cas de la croissance de diamant dopé au bore, la source de bore est par exemple le triméthylbore (B(CH₃)₃) ou le diborane (B₂H₆). Le dépôt est réalisé à une température comprise typiquement entre 500 et 1000°C.

Bien évidemment, afin d'initier la croissance d'un film de diamant dopé, des particules de diamant de tailles nanométriques (de préférence, une taille comprise entre 5 et 100 nm) sont disposées aux endroits où l'on souhaite obtenir des électrodes, c'est-à-dire sur la totalité de la face supérieure du substrat pour former un film continu dans le cas où le moule est obtenu par structuration d'une couche de poudre de diamant, ou localisé sur les parois des cavités du moule, dans le cas où le moule est déjà structuré. Les particules de diamant sont disposées selon une densité typiquement comprise entre 10⁸ et 10¹¹ particules par centimètre carré, en utilisant des techniques bien connues de l'homme du métier de la croissance diamant, comme par exemple la nucléation assistée par polarisation ou par trempage dans une solution colloïdale de particules de diamant de taille nanométrique. La croissance de ces grains de diamant par une technique CVD conduira à l'obtention d'un film de diamant polycristallin.

Les lignes d'interconnexion permettent d'obtenir une prise de contacts entre les électrodes en diamant dopé et l'instrumentation électronique fournissant l'énergie. Ces lignes d'interconnexion sont des pistes en matériau électriquement conducteur déposées localement sur les électrodes et la couche en premier matériau électriquement isolant, puis encapsulées, là où cela est nécessaire, dans une couche de matériau électriquement isolant, qui est de préférence le même matériau électriquement isolant que celui utilisé pour la première couche de matériau électriquement isolant. Le matériau électriquement conducteur est de préférence choisi parmi le platine, l'or, le titane ou tout autre métal susceptible de fournir un contact électrique de bonne qualité entre l'électrode et l'instrumentation, sans se dégrader dans le temps. Bien que ces métaux soient encapsulés, il est préférable de choisir des métaux qui soient biocompatibles. Le dépôt des lignes d'interconnexion est réalisé par des technologies de salle blanche connues de l'homme du métier.

Le procédé selon l'invention permet de fabriquer un implant rétinien intraoculaire comprenant à la fois un substrat souple et des électrodes en diamant dopé. Le fait que le substrat soit souple permet d'obtenir une implantation peu invasive, ainsi qu'un meilleur contact avec les tissus en épousant au mieux la forme de la rétine. L'obtention d'un meilleur contact permet entre autres d'utiliser de plus faibles niveaux de courants de stimulation, ce qui conduit au final à une augmentation de la résolution géométrique de l'implant.

L'implant réalisé selon le procédé de l'invention peut être implanté sur ou sous la rétine durant plusieurs années sans créer de modification des tissus environnants (pas de formation de cellules gliales ou de nécrose des tissus...). Les performances de l'implant ne se dégradent pas dans le temps, évitant ainsi toute intervention ultérieure visant à changer ou à modifier l'implant.

Cet implant peut permettre de réaliser une rétine artificielle. Il est adapté à une implantation dans des tissus rétiniens vivants pour permettre, par exemple, la restauration de la vue chez certains patients atteints de dégénérescence des photorécepteurs.

Il est possible de réaliser des implants planaires dans lesquels les extrémités distales des électrodes sont sensiblement au même niveau que la surface de la plaque souple sur laquelle elles se trouvent ; il est également possible de réaliser des implants dans lesquels les électrodes présentent une structure tridimensionnelle et sont en saillie de la plaque souple sur laquelle elles sont disposées, ce qui leur permet d'atteindre plus facilement les zones d'intérêt spécifiques dans le tissu rétinien et d'assurer un ancrage dans les tissus, évitant ainsi un déplacement de l'implant par glissement sur les tissus cellulaires de la rétine. Les structures tridimensionnelles des électrodes peuvent être toute forme allongée, comme par exemple une forme cylindrique, pyramidale, conique, parallélépipédique...

### BRÈVE DESCRIPTION DES DESSINS

L'invention sera mieux comprise et d'autres avantages et particularités apparaîtront à la lecture de la description qui va suivre, donnée à titre d'exemple non limitatif, accompagnée des dessins annexés parmi lesquels :
- les figures 1A à 1K représentent les étapes du procédé de l'invention selon une première variante permettant d'obtenir un implant planaire ;
- les figures 2A à 2I représentent les étapes du procédé de l'invention selon une seconde variante permettant d'obtenir un implant planaire ;
- les figures 3A à 3G représentent les étapes du procédé selon l'invention selon une troisième variante permettant de réaliser un implant comportant des électrodes tridimensionnelles.

### EXPOSÉ DÉTAILLÉ DE MODES DE RÉALISATION PARTICULIERS

Le procédé selon l'invention permet de réaliser un implant rétinien intraoculaire formé d'un ensemble d'électrodes en diamant dopé disposé sur un substrat souple.

Les électrodes sont de préférence des microélectrodes, c'est-à-dire des électrodes dont la plus grande dimension est comprise entre 1 et 1000 micromètres, de préférence entre 1 et 100 micromètres.

Dans le cas où les électrodes ont une forme allongée, par exemple une forme de colonne, on aura alors un diamètre inférieur à la dimension la plus grande, en l'occurrence la hauteur ; le diamètre sera donc lui aussi de valeur micrométrique ou inférieure.

Comme premier exemple de réalisation, nous allons décrire la fabrication d'un implant planaire à électrodes en diamant dopé au bore.

Afin d'obtenir une croissance localisée du diamant dopé sur le substrat, on réalise le moule en utilisant le procédé de structuration divulgué dans le document **[4]**, qui consiste à structurer une couche de particules de diamant déposée sur la face d'un substrat. Pour obtenir la structuration de la couche de diamant, on peut utiliser l'une quelconque des techniques divulguées dans ce document **[4]**.

Par exemple, pour réaliser un moule 100, on dépose, sur la face supérieure d'un substrat 1 de silicium, une couche sacrificielle 2, par exemple une couche d'oxyde de silicium ayant une épaisseur de 1,5 µm. Cette couche sacrificielle aidera au retrait du substrat à la fin du procédé de fabrication de l'implant. On précise cependant que cette couche sacrificielle 2 n'est pas essentielle et peut tout à fait être omise.

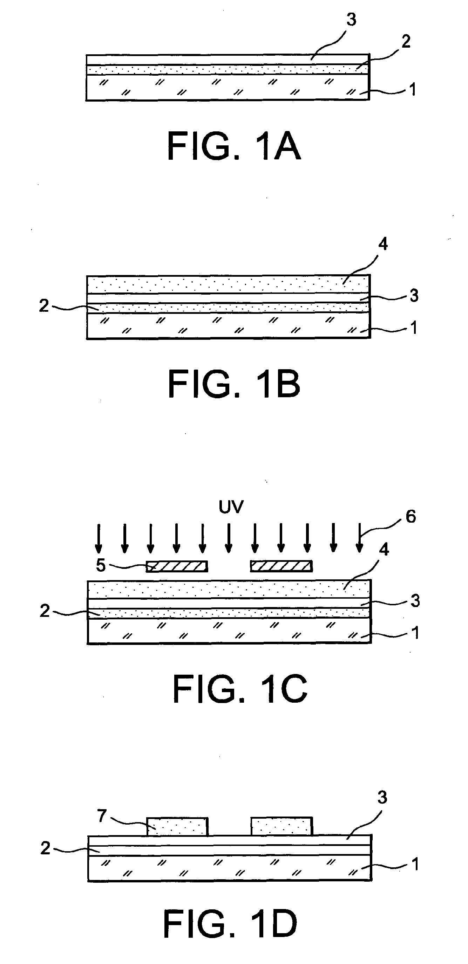

Puis, une couche de particules de diamant 3 est déposée sur la couche sacrificielle 2 (figure 1A) de façon à obtenir une densité de particules de diamant d'environ 10¹⁰ à 10¹¹ particules par cm². Les particules de diamant peuvent par exemple être des particules de la nano-poudre SYNDIA^{®} fournie par la société Van Moppes, en Suisse, ayant la référence 11247 ; cette poudre possède les caractéristiques suivantes : 14,8 carats/kg et un « grade 0-0.02 GAF » en anglais, c'est-à-dire que la poudre de diamant est conditionnée sous forme de diamant liquide (elle est en solution et non sous forme sèche) et est garantie exempte d'agglomérats (GAF pour « guaranteed agglomerate free » en anglais), avec des particules de tailles comprises entre 0 et 0,02 µm.

Ensuite, une couche de résine photosensible 4 positive ou négative est déposée sur la couche de nano-poudre de diamant 3 (figure 1B).

Cette couche de résine photosensible 4 est ensuite insolée de manière sélective par un rayonnement électromagnétique 6 (par exemple des rayons UltraViolets (UV)) à travers un masque 5 (figure 1C) dont les motifs sont choisis en fonction des motifs de la microstructuration que l'on souhaite donner à la future couche de diamant.

La couche de résine photosensible 4 est ensuite développée selon une technique connue, afin d'obtenir une couche de résine photosensible microstructurée 7 sur le substrat (figure 1D).

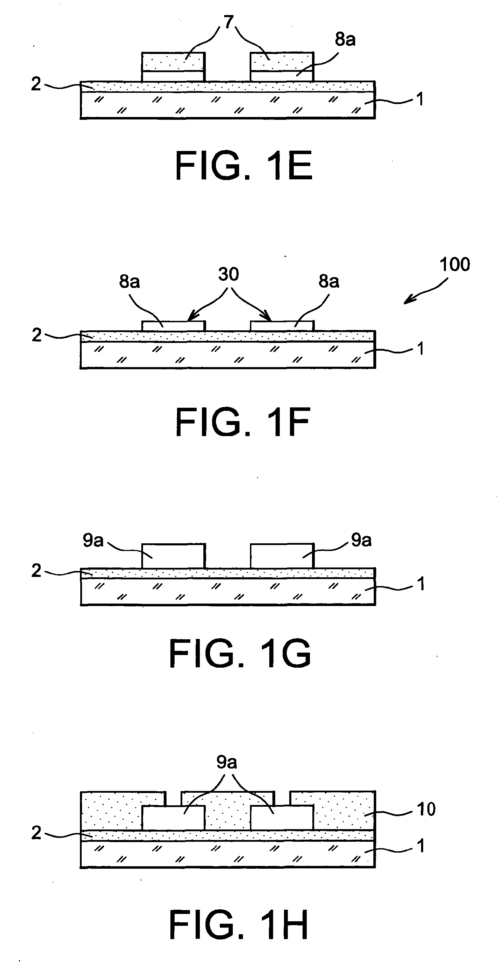

L'étape suivante consiste à enlever les zones de la couche de nano-poudre de diamant 3 qui ne sont pas recouvertes (masquées) par la couche de résine microstructurée 7, pour se retrouver avec une couche de nano-poudre de diamant structurée 8a. Pour cela, le substrat muni de ses différentes couches est par exemple placé dans un environnement plasma contenant un gaz ou un mélange de gaz permettant d'effectuer la gravure de la couche de nano-poudre de diamant 3 (figure 1E). Le gaz utilisé pour la gravure peut être par exemple SF₆, ou encore un mélange adéquat d'oxygène et d'argon.

La couche de résine microstructurée 7 est alors enlevée à l'aide d'une solution liquide adéquate, par exemple avec de l'acide nitrique concentré : on obtient alors un moule 100 comportant un substrat 1 dont une face est munie d'une couche de nano-poudre de diamant structurée 8a (figure 1F).

Enfin, une couche de diamant microstructurée 9a est mise à croitre sur la couche de nano-poudre de diamant microstructurée (figure 1G).

Dans ce mode de réalisation, on constate que ce sont les éléments en relief 30 du moule qui vont former les électrodes de diamant dopé lors de la transformation de la nano-poudre de diamant en matériau de diamant dopé (transformation des éléments 8a en éléments 9a).

Il existe plusieurs méthodes pour synthétiser une couche de diamant. Les méthodes les plus utilisées sont les méthodes de dépôt chimique à partir d'une phase vapeur assistée par plasma (ou méthode de croissance de type PECVD, pour « Plasma Enhanced Chemical Vapour Deposition » en anglais, le plasma pouvant être dans la gamme des micro-ondes (MPCVD) ou des Radio-Fréquence (RFCVD)) ou les méthodes de dépôt chimique à partir d'une phase vapeur assistée par filament chaud.

Dans notre mode de réalisation, on peut par exemple placer le dispositif dans un réacteur de croissance de type MPCVD. La méthode MPCVD consiste à faire croitre des grains de diamant de taille nanométrique sur un substrat placé dans un réacteur de croissance MPCVD à 2,45 GHz, fonctionnant typiquement entre 0,5 et 6 kilowatts, alimenté avec un mélange gazeux comprenant au moins un mélange de méthane et de dihydrogène en proportion adéquate (c'est-à-dire une proportion de méthane comprise entre 0,1 et 5%). Dans notre exemple, les conditions de croissance du diamant dopé sont les suivantes :
- une puissance micro-onde de 1000 watts ;
- un mélange de gaz comprend 99% d'hydrogène, 1% de méthane et 100 ppm de triméthylbore, le mélange étant à une pression de 50 mBar ;
- le substrat est mis à chauffer à une température de 700°C.

Au bout de 20 heures, la croissance des électrodes de diamant dopé au bore est stoppée.

On effectue ensuite un premier dépôt d'une couche de polyimide 10 sur la face supérieure du substrat 1 en recouvrant les électrodes 9a. Le dépôt peut être réalisé à la tournette (« spin coating » en anglais). Le polyimide peut, par exemple, être la référence PI 2611 de chez Dupont.

La couche de polyimide 10 subit ensuite un recuit à 350°C sous azote.

Cette couche de polyimide 10 laisse apparentes les électrodes 9a (figure 1H). Pour cela, la couche de polyimide 10 peut par exemple être photolithographiée : une couche d'un matériau quelconque pouvant servir de masque est déposée sur la totalité de la face supérieure du substrat (par exemple, on peut déposer une couche de métal comme l'aluminium par PVD (« Plasma Vapour Déposition » en anglais)), puis une étape de photolithographie est ensuite utilisée afin de définir la forme de l'implant (ses contours) et pour prendre les contacts sur les micro-électrodes en formant des lignes d'interconnexion.

Une fois la couche d'aluminium gravée, par exemple par plasma d'un mélange Cl₂ / BCl₃ / Ar, le polyimide est à son tour gravé par plasma dans un mélange O₂ / Ar.

La couche d'aluminium servant de masquage est ensuite éliminée dans un bain d'acide orthophosphorique à 60°C.

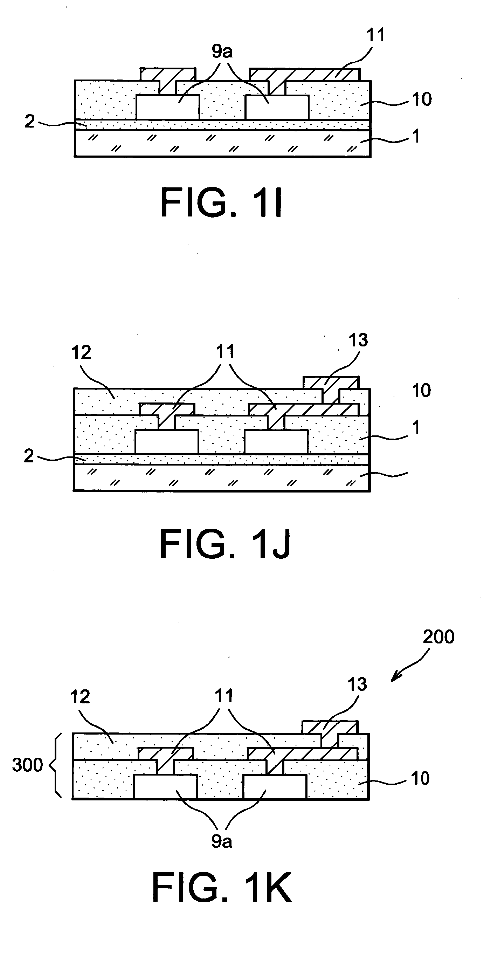

Enfin, des lignes d'interconnexion 11 sont formées (figure 1I) ; ces lignes vont permettre de relier les électrodes 9a au système électronique (non représenté) qui va fournir l'énergie électrique. Pour cela, un dépôt de titane et d'or est réalisé sur la couche de polyimide et sur les électrodes en diamant dopé. Ces lignes d'interconnexion 11 peuvent être obtenues par une étape de photolithographie.

Une seconde couche 12 de polyimide est réalisée comme précédemment. Il s'agit ici avec cette seconde couche 12 d'isoler électriquement toutes les lignes d'interconnexion 11 du milieu extérieur.

On dépose sur la seconde couche 12 de polyimide une couche d'aluminium qui va servir de masque de gravure, puis on grave cette couche de polyimide, par exemple par plasma Ar/O₂, à certains endroits jusqu'à atteindre certaines lignes d'interconnexion afin de former des plots de contact 13 qui serviront à connecter les électrodes au système électronique délivrant les stimulations électriques.

La couche d'aluminium est ensuite dissoute comme précédemment dans un bain d'acide orthophosphorique (figure 1J).

L'implant 200 est ensuite décollé du moule 100 en plaçant le substrat 1 dans un bain d'acide fluorhydrique qui va graver la couche sacrificielle 2 d'oxyde de silicium (figure 1K). L'implant obtenu comprend bien une plaque souple 300 (formée de la première 10 et de la seconde 12 couche électriquement isolante) dont une face comporte des électrodes 9a en diamant dopé.

L'implant une fois décollé est ensuite rincé et séché ; il sera stérilisé avant d'être implanté chez un patient.

Il est à noter qu'il est tout à fait possible de réaliser plusieurs implants en même temps sur un même substrat.

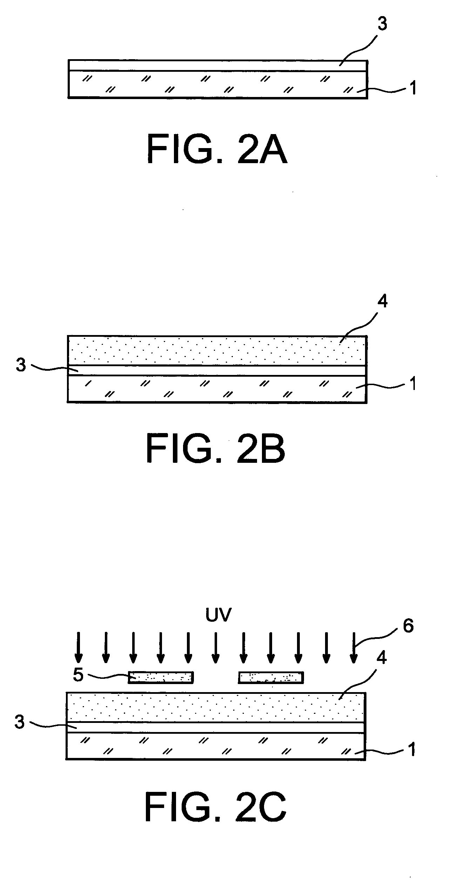

D'autres variantes de réalisation sont possibles. Par exemple, une autre technique divulguée dans le document le document **[4]** consiste à déposer, sur la face supérieure d'un substrat 1 de silicium, une couche de particules de diamant 3 (figure 2A) de façon à obtenir, comme dans l'exemple précédent, une densité de particules de diamant d'environ 10¹⁰ à 10¹¹ particules par cm². On précise que dans cet exemple, nous n'avons pas déposé de couche sacrificielle entre le substrat et la couche de nanopoudre de diamant, mais il est tout à fait possible de le faire, comme dans l'exemple précédent.

Ensuite, une couche de résine photosensible 4 positive ou négative est déposée sur la couche de nano-poudre de diamant 3 (figure 2B).

Cette couche de résine photosensible 4 est ensuite insolée de manière sélective par un rayonnement électromagnétique 6 à travers un masque 5 (figure 2C).

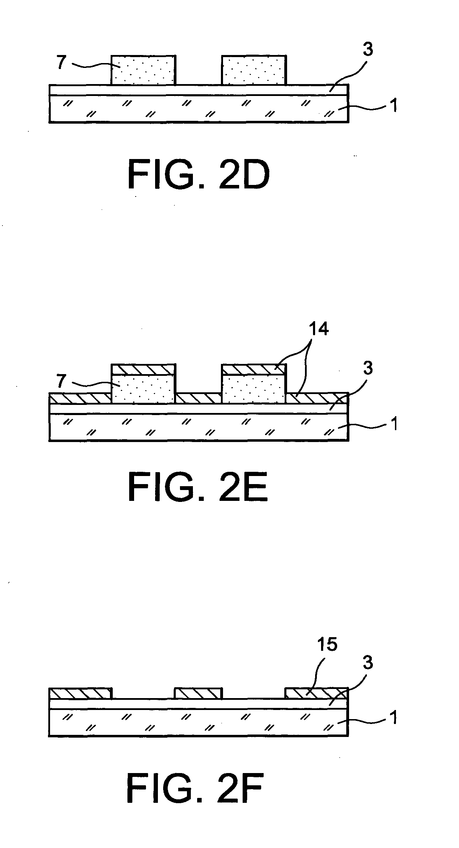

La couche de résine photosensible 4 est ensuite développée selon une technique connue, afin d'obtenir une couche de résine photosensible microstructurée 7 sur le substrat (figure 2D).

L'étape suivante consiste à déposer une couche métallique 14 (par exemple une couche d'aluminium) sur la surface (figure 2E), puis d'enlever la résine ainsi que les parties de la couche métallique situées en dessous de la couche de résine. On obtient ainsi, par procédé de lift-off, une couche métallique microstructurée 15 (figure 2F).

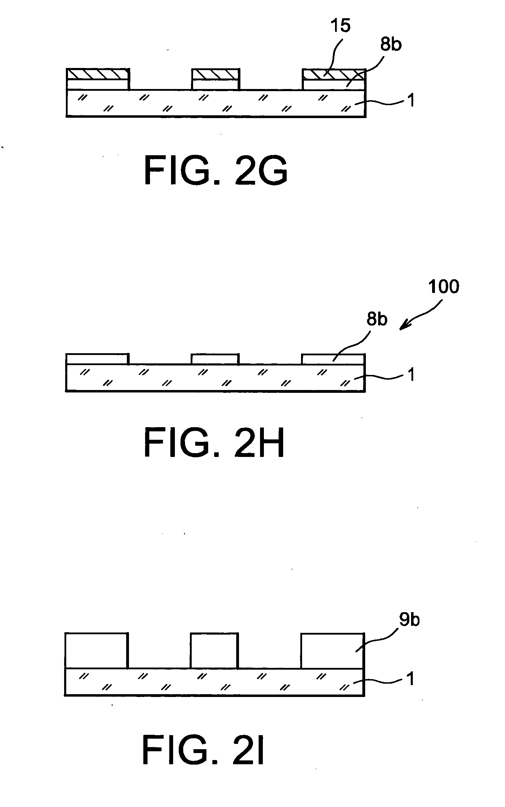

Ensuite, on enlève les zones de la couche de nano-poudre de diamant 3 qui ne sont pas recouvertes (masquées) par la couche métallique microstructurée 15, pour se retrouver avec une couche de nano-poudre de diamant structurée 8b au dessous de la couche métallique (figure 2G).

La couche métallique structurée 15 est alors enlevée à l'aide d'une solution liquide adéquate, par exemple une solution de soude de concentration 5M: on obtient alors un moule 100 comportant un substrat 1 dont une face est munie d'une couche de nano-poudre de diamant structurée 8b (figure 2H).

Enfin, une couche de diamant microstructurée 9b est mise à croitre sur la couche de nano-poudre de diamant microstructurée 8b (figure 2I).

Dans ce mode de réalisation, ce sont aussi les éléments en relief du moule qui vont former les électrodes de diamant dopé lors de la transformation de la nano-poudre de diamant en matériau de diamant dopé (transformation des éléments 8b en éléments 9b).

On poursuit la réalisation de l'implant en réalisant des étapes similaires à celles présentées dans l'exemple précédent (figures 1H-1K) pour réaliser les couches de matériau électriquement isolant et les lignes d'interconnexion.

Comme dernier exemple de réalisation, nous allons décrire la fabrication d'un implant tridimensionnel dont les électrodes en diamant dopé sont en saillie par rapport à la surface de la plaque souple de l'implant.

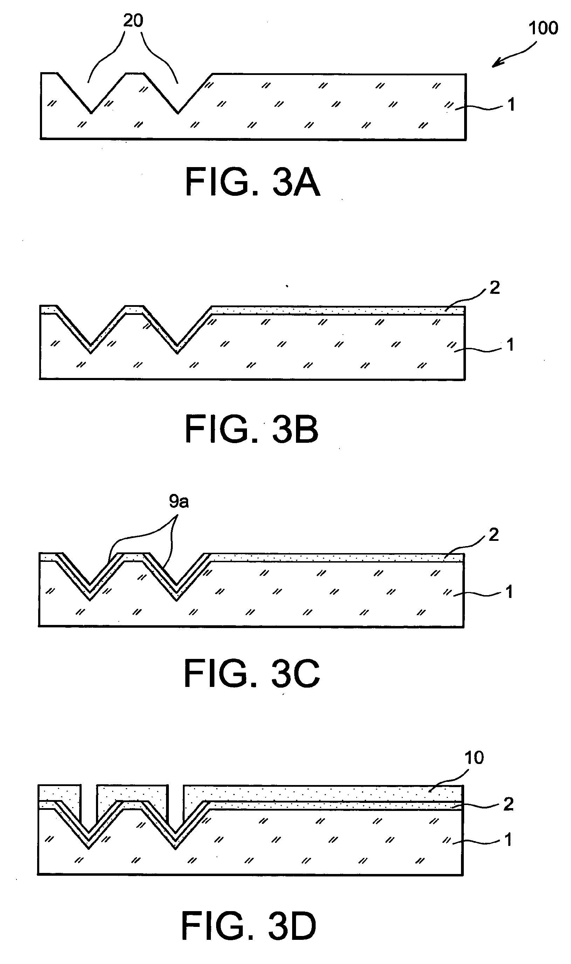

Le procédé de fabrication est similaire à celui décrit pour le premier exemple. Seule la préparation du moule 100 est différente. En effet, avant de procéder aux étapes décrites dans le premier exemple, on réalise des cavités 20 dans le substrat 1 (figure 3A). Ici, contrairement à l'exemple précédent, on constate que ce sont les éléments en creux du moule (les cavités 20) qui vont former les électrodes de diamant dopé : ces cavités forment l'empreinte des électrodes que l'on cherche à obtenir.

Un substrat 1 de silicium orienté (100), ayant de préférence une épaisseur supérieure à 300 micromètres, est oxydé de façon à obtenir une couche d'oxyde de silicium d'environ 400 nm d'épaisseur sur la face supérieure, cette couche servant de masque de gravure.

La couche d'oxyde de silicium est gravée localement grâce à une technique de photolithographie pour délimiter l'emplacement des futures électrodes.

Puis le substrat est placé dans un bain de KOH à 80°C.

En fonction du dessin du masque de gravure et de l'orientation cristallographique du substrat, on peut obtenir des cavités de formes différentes. Par exemple, lorsque des ouvertures de forme carrée sont réalisées dans la couche d'oxyde, la gravure anisotrope du substrat de silicium dans le bain de KOH donnera des cavités pyramidales. De même, en faisant varier le temps de gravure, des cavités plus au moins profondes sont obtenues.

Typiquement, la hauteur des électrodes tridimensionnelles sera comprise entre 1 et 100 micromètres ; la profondeur des cavités sera donc comprise dans ces mêmes valeurs.

Une fois que les cavités sont formées, le substrat est nettoyé.

Puis, une couche épaisse d'oxyde de silicium, par exemple une couche de 1,5 µm d'épaisseur, est déposée sur la face supérieure du substrat : elle servira de couche sacrificielle 2. On précise que l'ajout de cette couche sacrificielle est optionnel (figure 3B).

Puis, nous utilisons les mêmes étapes que celles décrites dans le premier exemple pour terminer l'implant tridimensionnel.

On dépose ainsi une couche de particules de diamant dans les endroits où l'on souhaite obtenir les électrodes de diamant, à savoir sur les parois des cavités du moule - on procède à ce que l'on appelle, en anglais, un « nanoseeding » - puis, on réalise la croissance de la couche de diamant 9a (figure 3C). Il est à noter qu'il est possible de former une couche de diamant uniquement sur les parois des cavités ou, au contraire, dans tout l'espace occupé par les cavités. Dans ce dernier cas, on stoppera la croissance du diamant lorsque la totalité de l'espace des cavités du moule sera rempli de diamant dopé.

Puis, une couche de matériau électriquement isolant et biocompatible 10 (par exemple un polymère) est déposée sur la face du moule comportant les électrodes afin de recouvrir toute la structure tridimensionnelle de la face du moule, y compris les électrodes 9a.

Une gravure locale est ensuite réalisée afin d'ouvrir des contacts sur les électrodes 9a (figure 3D).

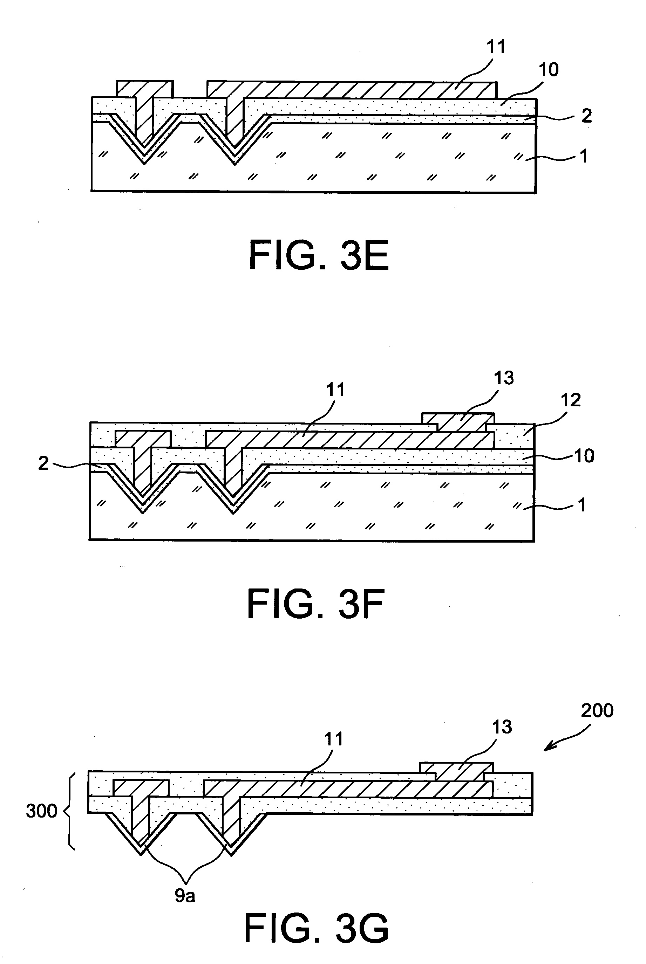

Les lignes d'interconnexion 11 sont ensuite réalisées par dépôt standard (PDV ou évaporation) d'un matériau électriquement conducteur, par exemple un métal, et par photolithographies (figure 3E).

Une seconde couche12 de matériau électriquement isolant et biocompatible, par exemple un polymère et, de préférence, le même matériau utilisé pour la première couche, est déposée sur la structure et gravée pour définir la forme de l'implant (figure 3F).

On peut réaliser des contacts 13 qui permettront de relier les lignes d'interconnexion 11 à une source de courant. Les ouvertures pour la formation de ces contacts peuvent être réalisées lors de la gravure de la première couche de matériau électriquement isolant ou lors de la gravure de la seconde couche de matériau électriquement isolant, c'est-à-dire au cours de l'étape 3D ou 3F.

Enfin, la couche sacrificielle 2 est gravée par voie chimique, ce qui a pour effet de détacher l'implant 200 du moule 100 (figure 3G).

### BIBLIOGRAPHIE

[1] WO 2007/148038 A1.
[2] K. Hungar and al., « Production processes for a flexible retina implant », Sensors and Actuators A, Physical, 123-124, 2005, pg 172-178. "WO 2007/148038 A1.
[3] P. Bergonzo, « Des diamants pour la vue », CEA Techno, N°89, juin 2008, pg 6.
[4] FR 08 55128

## Revendications

1. Procédé de fabrication d'un implant (200) rétinien intraoculaire, destiné à stimuler des cellules de la rétine par l'envoi d'impulsions électriques pour produire une vision artificielle, ledit implant comprenant une plaque souple (300) en un matériau biocompatible et électriquement isolant, qui est munie, sur l'une de ses faces, d'une pluralité d'électrodes en diamant dopé (9a) espacées les unes des autres et reliées à des lignes d'interconnexion (11) destinées à conduire un courant électrique jusqu'aux électrodes pour qu'elles puissent transmettre des impulsions électriques auxdites cellules de la rétine, ledit procédé comprenant les étapes successives suivantes :
- fourniture d'un moule (100) apte à supporter la croissance d'une couche de diamant dopé, ledit moule comportant, sur l'une de ses faces, un ensemble d'éléments, qui sont tous en creux (20) ou tous en saillie (30) par rapport à la surface de ladite face, et qui forment une empreinte des électrodes (9a) de l'implant que l'on souhaite obtenir ;
- réalisation des électrodes en diamant dopé (9a) par croissance d'une couche de diamant dopé dans tout ou partie de l'espace occupé par les éléments de l'empreinte ;
- formation d'une première couche (10) de matériau électriquement isolant sur la face du moule comportant l'empreinte, ladite première couche recouvrant au moins l'espace entre les électrodes (9a), tout en laissant découverte au moins une partie de la surface de chaque électrode ;
- réalisation des lignes d'interconnexion (11) par dépôt d'un matériau électriquement conducteur au moins dans les espaces non recouverts par la première couche (10) de matériau électriquement isolant ;
- formation d'une seconde couche (12) de matériau électriquement isolant sur la face du moule comprenant l'empreinte, ladite seconde couche recouvrant les lignes d'interconnexion (11) et la première (10) et la seconde (12) couche de matériau électriquement isolant formant la plaque souple (300) de l'implant ; et
- retrait du moule.

2. Procédé selon la revendication 1, dans lequel les matériaux de la première et de la seconde couche sont un même matériau.

3. Procédé selon la revendication 1, dans lequel le retrait du moule est obtenu par élimination chimique ou mécanique du moule.

4. Procédé selon la revendication 1, comprenant en outre, entre l'étape de fourniture d'un moule et l'étape de réalisation des électrodes, une étape de dépôt, sur la face du moule comportant l'empreinte, d'une couche sacrificielle supportant la croissance d'une couche de diamant dopé, ladite couche sacrificielle étant destinée à être retirée lors de l'étape de retrait du moule.

5. Procédé selon la revendication 4, dans lequel le retrait du moule est obtenu par dissolution de la couche sacrificielle ou en détachant la couche sacrificielle.

6. Procédé selon la revendication 1, dans lequel la plus grande étendue des électrodes mesurée dans une direction parallèle au plan de la face de la plaque souple comportant les électrodes est de dimension micrométrique ou inférieure.

7. Procédé selon la revendication 1, dans lequel la distance qui sépare deux électrodes adjacentes est de dimension micrométrique ou inférieure.

8. Procédé selon la revendication 1, dans lequel le diamant dopé est du diamant dopé au bore.

9. Procédé selon la revendication 1, dans lequel la fourniture du moule comprend les étapes successives suivantes :
- fourniture d'un substrat (1) apte à supporter la croissance d'une couche de diamant dopé sur une de ses faces ;
- dépôt d'une couche de poudre de diamant (3) sur ladite face du substrat, les grains de la poudre de diamant ayant une taille nanométrique ;
- dépôt d'une couche de résine photosensible (4) d'une épaisseur déterminée sur la couche de poudre de diamant ;
- structuration de la couche de poudre de diamant (3) par photolithographie de la couche de résine photosensible (4) pour obtenir l'empreinte du moule.

10. Procédé selon la revendication 1, dans lequel la fourniture du moule comprend les étapes successives suivantes :
- fourniture d'un substrat (1) apte à supporter la croissance d'une couche de diamant dopé sur une de ses faces ;
- dépôt d'une couche de poudre de diamant (3) sur ladite face du substrat, les grains de la poudre de diamant ayant une taille nanométrique ;
- dépôt d'une couche de résine photosensible (4) d'une épaisseur déterminée sur la couche de poudre de diamant ;
- structuration de la couche de résine photosensible (4) par photolithographie jusqu'à atteindre la couche de poudre de diamant (3) ;
- dépôt d'une couche métallique (14) sur la face du substrat (1), ladite couche métallique recouvrant la couche de résine structurée (7) et la couche de poudre de diamant (3) non masquée par la couche de résine structurée (7) ;
- retrait de la couche de résine structurée (7) de manière à former une couche métallique structurée (15) ;
- structuration de la couche de poudre de diamant (3) par gravure de la couche de poudre de diamant non masquée par la couche métallique structurée (15) ;
- retrait de la couche métallique structurée (15) pour obtenir l'empreinte du moule.

## Patentansprüche

1. Verfahren zur Herstellung eines intraokularen Retina-Implantats (200), das vorgesehen ist zum Stimulieren von Retina-Zellen durch Aussenden elektrischer Impulse, um ein künstliches Sichtsystem zu erzeugen, wobei das Implantat eine flexible Platte (300) auf einem biokompatiblen und elektrisch isolierenden Material umfasst, die auf einer der Oberflächen mit mehreren Elektroden (9a) aus dotiertem Diamant ausgestattet ist, die voneinander beabstandet sind und verbunden sind durch Verbindungsleitungen (11), die vorgesehen sind zum Leiten eines elektrischen Stroms bis zu den Elektroden, so dass diese elektrische Impulse der Retina-Zellen weiterleiten können, wobei das Verfahren die folgenden Schritte umfasst:
- Bereitstellen einer Form (100), angepasst zum Unterstützen des Wachstums einer dotierten Diamantenschicht , wobei die Form auf einer ihrer Oberflächen einen Satz von Elementen umfasst, die alle vertieft (20) oder vorspringend (30) sind bezüglich der Oberfläche, und welche einen Abdruck der Elektroden (9a) des gewünschten zu erhaltenden Implantats bilden;
- Herstellen von Elektroden (9a) aus dotiertem Diamant durch Wachstum einer Schicht aus dotiertem Diamant in dem gesamten oder einem Teil Raums , der durch die Abdruckelemente eingenommen wird;
- Bilden einer ersten Schicht (10) aus elektrisch isolierendem Material auf der Formoberfläche, die den Abdruck trägt, wobei die erste Schicht mindestens den Raum zwischen den Elektroden (9a) überdeckt, wobei zumindest ein Teil der Oberfläche jeder Elektrode freigelegt bleibt;
Herstellen der Verbindungsleitungen (11) durch Abscheidung eines elektrisch leitenden Materials, zumindest in den Bereichen, die nicht durch die erste Schicht (10) bedeckt sind, aus elektrisch isolierendem Material;
Bilden einer zweiten Schicht (12) aus einem elektrisch isolierendem Material auf der Oberfläche der Form, die den Abdruck umfasst, wobei die zweite Schicht die Verbindungsleitungen (11) und die erste (10) und zweite (12) Schicht aus elektrisch isolierendem Material überdeckt, das die flexible Platte (300) des Implantats bildet; und Entfernen der Form

2. Verfahren nach Anspruch 1, worin die Materialien der ersten und zweiten Schicht aus gleichem Material sind.

3. Verfahren nach Anspruch 1, worin das Entfernen der Form erreicht wird durch chemische oder mechanische Eliminierung der Form.

4. Verfahren nach Anspruch 1, weiterhin umfassend zwischen dem Schritt der Bereitstellung einer Form und der Herstellung der Elektroden einen Ablagerungsschritt auf der Oberfläche der Form, die den Abdruck umfasst, einer Opferschicht, die das Wachstum einer dotierten Diamantschicht unterstützt, worin die Opferschicht dafür vorgesehen ist während des Schritts des Entfernens der Form ausgesondert zu werden.

5. Verfahren nach Anspruch 4, worin das Entfernen der Form erreicht wird durch Auflösen der Opferschicht oder ablösen der Opferschicht.

6. Verfahren nach Anspruch 1, worin die größte gemessene Ausdehnung der Elektroden in einer Richtung parallel zur Ebene der Oberfläche der flexiblen Platte, die die Elektroden aufweist, im Mikrometerbereich oder kleiner ist.

7. Verfahren nach Anspruch 1, worin der Abstand, der zwei benachbarte Elektroden trennt, im Mikrometerbereich oder kleiner ist.

8. Verfahren nach Anspruch 1, worin der dotierte Diamant mit Bor dotierter Diamant ist.

9. Verfahren nach Anspruch 1, worin die Bereitstellung der Form die folgenden aufeinanderfolgenden Schritte umfasst:
- Bereitstellung eines Substrats (1) , das angepasst ist zum Unterstützen des Wachstums einer Schicht aus dotiertem Diamant auf einer seiner Oberflächen;
- Abscheidung einer Diamantpulverschicht (3) auf der Oberfläche des Substrats, wobei die Körner des Diamantpulvers eine Nanometer-Größe haben;
- Abscheidung einer Schicht (4) aus lichtempfindlichem Harz in einer vorbestimmten Dicke auf der Schicht aus Diamantpulver;
- Strukturierung der Schicht (3) aus Diamantpulver durch Photolithographie der Schicht (4) aus lichtempfindlichem Harz zum Erhalten des Abdrucks der Form.

10. Verfahren nach Anspruch 1, worin die Bereitstellung der Form die folgenden aufeinanderfolgenden Schritte umfasst:
- Bereitstellung eines Substrats (1), das angepasst ist zum Unterstützen des Wachstums einer Schicht aus dotiertem Diamant auf einer seiner Oberflächen;
- Abscheidung einer Diamantpulverschicht (3) auf der Oberfläche des Substrats, wobei die Körner des Diamantpulvers eine Nanometer-Größe haben;
- Abscheidung einer Schicht (4) aus lichtempfindlichem Harz in einer vorbestimmten Dicke auf der Schicht aus Diamantpulver;
- Strukturierung der Schicht (4) aus lichtempfindlichem Harz durch Photolithographie bis zum Erhalten einer Schicht (3) aus Diamantpulver; Abscheidung einer metallischen Schicht (14) auf der Oberfläche des Substrats (1), wobei die metallische Schicht die Schicht (7) aus strukturiertem Harz und die Schicht (3) aus Diamantpulver, die nicht maskiert ist durch die Schicht (7) aus strukturiertem Harz, überdeckt;
- Entfernen der Schicht (7) aus strukturiertem Harz, um eine strukturierte metallische Schicht (15) zu bilden;
- Strukturieren der Schicht aus Diamantpulver (3) durch Gravieren der nichtmaskierten Schicht aus Diamantpulver durch die strukturierte metallische Schicht (15);
- Entfernen der strukturierten metallischen Schicht (15), um den Abdruck der Form zu erhalten.

## Claims

1. Method for manufacturing an intraocular retinal implant (200), intended to stimulate the cells of the retina by sending electrical impulses to produce an artificial vision, said implant including a flexible plate (300) made of a biocompatible and electrically insulting material, which is provided, on one of the face thereof, with a plurality of electrodes made of doped diamond (9a) spaced apart from each other and connected to interconnection lines (11) intended to lead an electric current to the electrodes so that they can transmit electrical impulses to said cells of the retina, said method including the following successive steps:
- providing a mould (100) capable of supporting the growth of a layer of doped diamond, said mould comprising, on one of the face thereof, a set of elements, which are all depressed (20) or all projecting (30) with respect to the surface of said face, and which constitute a pattern cavity for the electrodes (9a) of the implant which it is desired to obtain;
- producing electrodes made of doped diamond (9a) by growing a layer of doped diamond in all or part of the space occupied by the elements of the pattern cavity;
- forming a first layer (10) of electrically insulating material on the face of the mould comprising the pattern cavity, said first layer covering at least the space between the electrodes (9a), while leaving uncovered at least a part of the surface of each electrode;
- producing interconnection lines (11) by depositing an electrically conductive material at least in the spaces not covered by the first layer (10) of electrically insulating material;
- forming a second layer (12) of electrically insulating material on the face of the mould including the pattern cavity, said second layer covering the interconnection lines (11) and the first (10) and the second (12) layers of electrically insulating material forming the flexible plate (300) of the implant; and
- removing the mould.

2. Method according to claim 1, wherein the materials of the first and the second layers are a same material.

3. Method according to claim 1, wherein removing the mould is obtained by chemical or mechanical elimination of the mould.

4. Method according to claim 1, further including, between the step of providing a mould and the step of producing the electrodes, a step of depositing, on the face of the mould comprising the pattern cavity, a sacrificial layer supporting the growth of a layer of doped diamond, said sacrificial layer being intended to be lifted off during the step of removing the mould.

5. Method according to claim 4, wherein removing the mould is obtained by dissolution of the sacrificial layer or by detaching the sacrificial layer.

6. Method according to claim 1, wherein the greatest extent of the electrodes measured in a direction parallel to the plane of the face of the flexible plate comprising the electrodes is of micrometric dimension or less.

7. Method according to claim 1, wherein the distance that separates two adjacent electrodes is of micrometric dimension or less.

8. Method according to claim 1, wherein the doped diamond is diamond doped with boron.

9. Method according to claim 1, wherein the provision of the mould includes the following successive steps:
- providing a substrate (1) capable of supporting the growth of a layer of doped diamond on one of the faces thereof;
- depositing a layer of diamond powder (3) on said face of the substrate, the grains of the diamond powder having a nanometric size;
- depositing a layer of photosensitive resin (4) of a determined thickness on the layer of diamond powder;
- structuring the layer of diamond powder (3) by photolithography of the layer of photosensitive resin (4) to obtain the pattern cavity of the mould.

10. Method according to claim 1, wherein the provision of the mould includes the following successive steps:
- providing a substrate (1) capable of supporting the growth of a layer of doped diamond on one of the faces thereof;
- depositing a layer of diamond powder (3) on said face of the substrate, the grains of the diamond powder having a nanometric size;
- depositing a layer of photosensitive resin (4) of a determined thickness on the layer of diamond powder;
- structuring the layer of photosensitive resin (4) by photolithography until the layer of diamond powder (3) is reached;
- depositing a metal layer (14) on the face of the substrate (1), said metal layer covering the structured resin layer (7) and the layer of diamond powder (3) not masked by the structured resin layer (7);
- removing the structured resin layer (7) so as to form a structured metal layer (15);
- structuring the layer of diamond powder (3) by etching the layer of diamond powder not masked by the structured metal layer (15);
- removing the structured metal layer (15) to obtain the pattern cavity of the mould.
